(19) Europäisches Patentamt
European Patent Office
Office européen des brevets

(11) Veröffentlichungsnummer : **0 023 730**
**B1**

(12) # EUROPÄISCHE PATENTSCHRIFT

(45) Veröffentlichungstag der Patentschrift : **03.11.82**

(21) Anmeldenummer : **80200541.3**

(22) Anmeldetag : **12.06.80**

(51) Int. Cl.³ : **C 07 C 69/75**, C 07 C 79/22, C 07 C 67/08, C 07 C 69/76, C 09 K 3/34, C 07 C121/75, C 07 C 67/14, C 07 C101/18, C 07 C121/46, C 07 C121/64, C 07 D295/00

(54) **Cyclohexylcyclohexanoate, deren Herstellungsverfahren und diese enthaltende Flüssigkristallmischung.**

(30) Priorität : **18.07.79 CH 6672/79**

(43) Veröffentlichungstag der Anmeldung : **11.02.81 (Patentblatt 81/06)**

(45) Bekanntmachung des Hinweises auf die Patenterteilung : **03.11.82 Patentblatt 82/44**

(84) Benannte Vertragsstaaten : **CH DE FR GB LI NL**

(56) Entgegenhaltungen :
**CHEMICAL ABSTRACTS, Band 92, no. 18, 5. Mai 1980, Seite 625, Zusammenfassung 156105y COLUMBUS, Ohio (US) M.A. OSMAN et al. : « trans, trans-Cyclohexyl cyclohexanoates. A new class of aliphatic liquid crystals »**

(73) Patentinhaber : **Merck Patent Gesellschaft mit beschränkter Haftung**
**Frankfurter Strasse 250**
**D-6100 Darmstadt (DE)**

(72) Erfinder : **Osman, Maged A., Dr.**
**Lerchenrain**
**CH-8046 Zürich (CH)**
Erfinder : **Révész, Laszlo, Dr.**
**Niederrohrdorferstrasse 2**
**CH-5442 Fislisbach (CH)**

**EP 0 023 730 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

## Cyclohexylcyclohexanoate, deren Herstellungsverfahren und diese enthaltende Flüssigkristallmischung

Die Erfindung betrifft neue anisotrope Verbindungen, und zwar die neue Klasse der Cyclohexylhexanoate, sowie Flüssigkristall- (FK) mischungen, die Cyclohexylhexanoate enthalten und als Dielektrikum für Flüssigkristallanzeigen verwendet werden. Die Erfindung betrifft ferner ein Verfahren zur Herstellung der neuen anisotropen Verbindungen und FK-Mischungen, welche die neuen anisotropen Verbindungen enthalten.

Anisotrope Biphenylverbindungen der Formel (10)

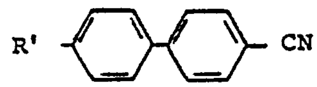

(10)

in der R' z.B. einen Alkylrest bedeutet, sind z.B. aus der DE-OS 2 356 085 bekannt und gehören zu den für FK-Mischungen am meisten verwendeten Substanzen.

Aus der DE-OS 2 636 684 ist ferner bekannt, dass sich die vergleichsweise hohen Viskositäts- und Δn-Werte der Verbindungen (10) vermindern lassen, wenn der eine Phenylenring durch einen trans-1,4-Cyclohexylenring ersetzt wird, was zu Phenylcyclohexanen der Formel (20)

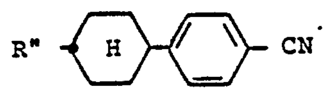

(20)

führt, in der R" die gleiche Bedeutung wie R' in Formel (10) hat.

Die Δn-Werte von anisotropen Komponenten von FK-Mischungen stellen bekanntlich ein Mass für die optische Anisotropie dar und werden als Differenz der Brechungsindizes parallel bzw. senkrecht zur Molekülachse der in Frage stehenden Substanz als $\Delta n = n_{\parallel} - n_{\perp}$ ausgedrückt.

Für manche Typen von FK-Anzeigen, insbesondere die sogenannten « Guest-host »-Effektzellen, werden nematische FK-Phasen mit möglichst kleinen Δn-Werten, z.B. nicht über 0,1, benötigt. Durch den Ersatz des einen Phenylenringes in den Verbindungen (10) durch den trans-Cyclohexylenring wird der Δn-Wert praktisch auf die Hälfte reduziert (Δn ~ 0,22 bei Verbindungen der Formel (10), Δn ~ 0,12 bei Verbindungen der Formel (20)).

Wird nun auch der zweite Phenylenring der anisotropen Verbindungen (10) bzw. der verbleibende Phenylenring der Verbindungen (20) durch den Cyclohexylenring ersetzt, so gelangt man zu den aus der DE-OS 2 702 598 bekannten Cyclohexylcyclohexanen der Formel (30)

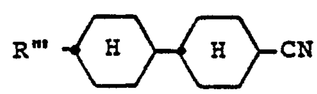

(30)

in der R‴ die für R' in Formel (10) angegebene Bedeutung hat ; wiederum wird hierdruch der Δn-Wert vermindert, und zwar auf Werte unter 0,1, typisch etwa auf Δn = 0,06.

Nachteilig bei den Verbindungen (30) ist aber die Tatsache (siehe R. Pohl et al, Phys. Letter *65A* (2), 169/1978), dass die Hydrierung des zweiten Phenylenringes eine ausgeprägte Tendenz zur Bildung von smektischen Phasen verursacht.

Aufgabe der Erfindung ist es, eine zur Verwendung in FK-Anzeigen geeignete Klasse neuer anisotroper Verbindungen anzugeben, die geringe Δn-Werte von vorzugsweise unter 0,1 besitzen und keine ausgeprägte Tendenz zur Bildung smektischer Phasen haben.

Es wurde gefunden, dass dieses Ziel dadurch erreicht wird, dass man die Planarität des Moleküls mit zwei einander benachbarten Cyclohexanringen durch die Zwischenschaltung der Carboxylgruppe zwischen die beiden Cyclohexanringe unterbricht, und zwar ohne die Anisotropie des Moleküls zu zerstören.

Die neuen anisotropen Verbindungen gemäss der Erfindung haben die Formel (1)

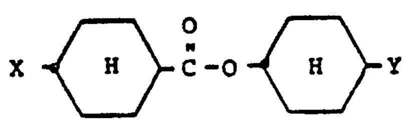

trans trans

(1)

in der X und Y jeweils Alkylgruppen mit 1 bis 12 C-Atomen, Alkoxygruppen mit 1 bis 12 C-Atomen, N-Monoalkylaminogruppen mit 1 bis 12 C-Atomen im Alkylteil, Halogenatome, Nitril- oder Nitrogruppen sind ; eine der Gruppen X, Y kann ferner einen cyclischen Rest der Formeln

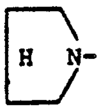 , 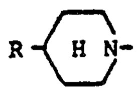 , 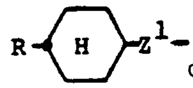 oder 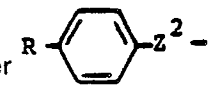

trans

bedeuten, in welchen R das Wasserstoffatom, ein Halogenatom, die Nitril- oder Nitrogruppe oder eine Alkyl- oder Alkoxygruppe mit 1 bis 12 C-Atomen, $Z^1$ die Gruppe —COO— oder —OOC— und $Z^2$ gleich $Z^1$ ist oder eine einfache Kovalenzbindung darstellt. Dabei darf aber nur eine der Gruppen X, Y einen cyclischen Rest bedeuten.

Die neuen anisotropen Verbindungen (1) unterscheiden sich von den aus DE-OS 2 429 093 bekannten Cyclohexancarbonsäurephenylester nicht nur in struktureller, sondern auch in verwendungstechnischer Hinsicht, insbesondere bezüglich der Δn-Werte.

Die neuen Verbindungen können dadurch hergestellt werden, dass man eine entsprechende Cyclohexancarbonsäure der Formel (2)

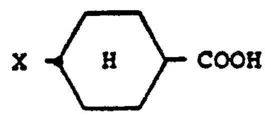

trans (2)

vorzugsweise nach Umwandlung in ein entsprechendes Säurehalogenid, mit dem entsprechenden Cyclohexanol der Formel (3)

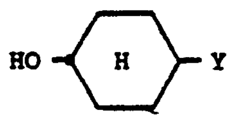

trans (3)

zur Zielverbindung (1) kondensiert.

Geeignete Cyclohexancarbonsäuren (2) bzw. Cyclohexanole (3) sind an sich bekannt bzw. können in an sich bekannter Weise hergestellt werden. Beispielsweise können die Verbindungen (2) durch Hydrieren der entsprechenden Benzoesäureverbindung erhalten werden.

Bei den Formeln (1), (2) und (3) handelt es sich bei den entsprechenden Verbindungen jeweils um die trans-Formen, wie in den Formeln durch Punkte in üblicher Weise angedeutet. Wenn z.B. bei der Herstellung der Ausgangsverbindungen (2), (3) cis-, trans-Isomerengemische entstehen, können die gewünschten trans-Isomeren in an sich üblicher Weise durch fraktionierte Kristallisation oder nach ähnlichen Methoden erhalten werden.

Vorzugsweise wird die Cyclohexancarbonsäureverbindung (2) mit $SO_2Cl$ in das entsprechende Säurehalogenid der Formel (2')

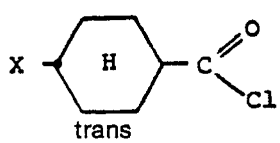

trans (2')

umgewandelt und dieses in Gegenwart einer normalerweise flüssigen organischen Base, die vorzugsweise auch als Reaktionsmedium dient, z.B. in Pyridin, mit der Cyclohexanolverbindung (3) kondensiert.

Die erfindungsgemässen neuen Verbindungen der Formel (1) sind anisotrop, d.h. entweder enantiotrop flüssigkristallin oder monotrop flüssigkristallin ; bei monotrop flüssigkristallinen Stoffen liegt der Klärpunkt ($T_c$) der reinen Substanz tiefer als deren Schmelzpunkt ($T_m$), kann aber z.B. durch Unterkühlung an der reinen monotropen Substanz gemessen oder durch Untersuchung einer die monotrope Substanz enthaltenden enantiotropen Mischung und Berechnung des Mesomorphiebeitrages der monotropen Substanz berechnet werden.

Ueberraschenderweise zeigen die neuen anisotropen Verbindungen der Formel (1) praktisch keine ausgeprägte Tendenz zu smektischen Phasen ; aufgrund der Eigenschaften der von R. Eidenschink et al (8. Freiburger Arbeitstagung, 1978) beschriebenen Cyclohexylester mit sehr breiten smektischen Phasen war vielmehr zu erwarten, dass eine zwischen zwei Cyclohexanringen stehende Carboxylgruppe die ausgeprägte Tendenz von Cyclohexylcyclohexanen (30) zur Bildung smektischer Mesophasen nicht wesentlich beeinflussen würde.

Andererseits hat die bei den neuen anisotropen Verbindungen (1) erfindungsgemäss als Brücke zwischen den benachbarten Cyclohexanringen verwendete Carboxylgruppe offenbar keine Wirkung auf die optische Anisotropie, da die erfindungsgemässen Verbindungen (1) Δn-Werte von unter 0,1 besitzen und dementsprechende Vorteile bei Verwendung als Komponenten von FK-Mischungen bieten.

Es versteht sich für den Fachmann aufgrund der obigen Darlegungen, dass die neuen anisotropen Verbindungen (1) ein ausserordentlich breites Anwendungsspektrum haben ; je nach Wahl der Substituenten X, Y in Formel (1) im Sinne einer stärkeren oder schwächeren Polarisierung des Moleküls können die neuen anisotropen Verbindungen (1) unterschiedliche Funktionen in FK-Mischungen für FK-Anzeigen unterschiedlicher Typen erfüllen ; dementsprechend lassen sich mit den anisotropen Verbindungen (1) — die einzeln oder in Mischung verwendet werden können — ganz unterschiedliche FK-Mischungen bilden, die überwiegend bzw. praktisch vollständig (z.B. bis 90 Gew.%) aus den neuen anisotropen Verbindungen (1) bestehen oder aber diese nur als Komponenten enthalten, z.B. in Anteilen von 2 bis 40 Gew.%. Als Halogen für X, Y und R werden meist Cl und Br bevorzugt.

Die folgenden Beispiele erläutern die Herstellung erfindungsgemässer Verbindungen der Formel (1). Prozentangaben sind auf das Gewicht bezogen. Wenn nicht anders vermerkt, liegen die als Ausgangsstoffe verwendeten Cyclohexanverbindungen in trans-Form vor. Alle nach den Beispielen erhaltenen Verbindungen (1) haben die trans-Konfiguration.

## Beispiel 1

Herstellung von 4-Methylcyclohexyl-4'-pentylcyclohexanoat (Formel 1 ; $X = H_{11}C_5$, $Y = CH_3$)

2,1 g (10,6 mMol) 4-n-Pentylcyclohexancarbonsäure (Formel 2 ; X wie oben) wurden in 10 ml Thionylchlorid während 20 min auf Rückfluss erwärmt, dann eingeengt, dreimal mit je 20 ml Toluol versetzt und wiederum eingeengt, um die letzten Reste Thionylchlorid zu vertreiben. Das Säurechlorid wurde in 12 ml Pyridin vorgelegt und unter Rühren mit 1,2 g (10,6 mMol) 4-Methylcyclohexanol in 6 ml Pyridin versetzt. Das Reaktionsgemisch wurde 2 Std. bei Raumtemperatur, 30 min bei 60 °C gerührt, auf Eis/2N HCl gegossen und dreimal mit Methylenchlorid extrahiert. Die organischen Phasen wurden einmal mit 2N NaOH/Eis, einmal mit NaCl-Lösung gewaschen, eingeengt und ergaben 3,0 g hellgelbe Kristalle. Nach Umkristallisation aus EtOH und Hexan wurde das reine Zielprodukt in Form von farblosen Kristallen (GC 99,7 %) erhalten, $T_m$ 18,3 °C, $T_c$ 3,3 °C.

## Beispiele 2-74

Nach der Arbeitsweise von Beispiel 1 wurden unter Verwendung der entsprechend substituierten Cyclohexancarbonsäureverbindung der Formel (2) mit den entsprechend substituierten Cyclohexanolen der Formel (3) die folgenden Verbindungen der Formel (1) hergestellt :

(2) 4-Propylcyclohexyl-4'-propylcyclohexanoat
(3) 4-Propylcyclohexyl-4'-pentylcyclohexanoat
(4) 4-Propylcyclohexyl-4'-heptylcyclohexanoat
(5) 4-Pentylcyclohexyl-4'-propylcyclohexanoat
(6) 4-Pentylcyclohexyl-4'-pentylcyclohexanoat
(7) 4-Propyloxycyclohexyl-4'-propylcyclohexanoat
(8) 4-Propyloxycyclohexyl-4'-pentylcyclohexanoat
(9) 4-Propyloxycyclohexyl-4'-heptylcyclohexanoat
(10) 4-Pentyloxycyclohexyl-4'-propylcyclohexanoat
(11) 4-Pentyloxycyclohexyl-4'-pentylcyclohexanoat
(12) 4-Propylcyclohexyl-4'-propyloxycyclohexanoat
(13) 4-Propylcyclohexyl-4'-pentyloxycyclohexanoat
(14) 4-Propylcyclohexyl-4'-heptyloxycyclohexanoat
(15) 4-Pentylcyclohexyl-4'-propyloxycyclohexanoat
(16) 4-Pentylcyclohexyl-4'-pentyloxycyclohexanoat
(17) 4-Propyloxycyclohexyl-4'-propyloxycyclohexanoat
(18) 4-Propyloxycyclohexyl-4'-pentyloxycyclohexanoat
(19) 4-Propyloxycyclohexyl-4'-heptyloxycyclohexanoat
(20) 4-Pentyloxycyclohexyl-4'-propyloxycyclohexanoat
(21) 4-Pentyloxycyclohexyl-4'-pentyloxycyclohexanoat
(22) 4-Pyrrolidinocyclohexyl-4'-nonylcyclohexanoat
(23) 4-Propylcyclohexyl-4'-cyanocyclohexanoat
(24) 4-Butylcyclohexyl-4'-cyanocyclohexanoat
(25) 4-Pentylcyclohexyl-4'-cyanocyclohexanoat
(26) 4-Nonylcyclohexyl-4'-cyanocyclohexanoat
(27) 4-Propylcyclohexyl-4'-nitrocyclohexanoat
(28) 4-Pentylcyclohexyl-4'-nitrocyclohexanoat
(29) 4-Nonylcyclohexyl-4'-nitrocyclohexanoat
(30) 4-Propylcyclohexyl-4'-chlorocyclohexanoat
(31) 4-Pentylcyclohexyl-4'-chlorocyclohexanoat
(32) 4-Nonylcyclohexyl-4'-chlorocyclohexanoat

(33) 4-Propylcyclohexyl-4'-bromocyclohexanoat
(34) 4-Pentylcyclohexyl-4'-bromocyclohexanoat
(35) 4-Nonylcyclohexyl-4'-bromocyclohexanoat
(36) 4-Cyanocyclohexyl-4'-propylcyclohexanoat
(37) 4-Cyanocyclohexyl-4'-pentylcyclohexanoat
(38) 4-Cyanocyclohexyl-4'-nonylcyclohexanoat
(39) 4-Nitrocyclohexyl-4'-propylcyclohexanoat
(40) 4-Nitrocyclohexyl-4'-pentylcyclohexanoat
(41) 4-Nitrocyclohexyl-4'-nonylcyclohexanoat
(42) 4-Chlorocyclohexyl-4'-propylcyclohexanoat
(43) 4-Chlorocyclohexyl-4'-pentylcyclohexanoat
(44) 4-Chlorocyclohexyl-4'-nonylcyclohexanoat
(45) 4-Bromocyclohexyl-4'-propylcyclohexanoat
(46) 4-Bromocyclohexyl-4'-pentylcyclohexanoat
(47) 4-Bromocyclohexyl-4'-nonylcyclohexanoat
(48) 4-Propylcyclohexyl-4'-butylaminocyclohexanoat
(49) 4-Propylcyclohexyl-4'-hexylaminocyclohexanoat
(50) 4-Pentylcyclohexyl-4'-butylaminocyclohexanoat
(51) 4-Pentylcyclohexyl-4'-hexylaminocyclohexanoat
(52) 4-Cyanocyclohexyl-4'-butylaminocyclohexanoat
(53) 4-Cyanocyclohexyl-4'-hexylaminocyclohexanoat
(54) 4-Nitrocyclohexyl-4'-butylaminocyclohexanoat
(55) 4-Nitrocyclohexyl-4'-hexylaminocyclohexanoat
(56) 4-Bromocyclohexyl-4'-butylaminocyclohexanoat
(57) 4-Bromocyclohexyl-4'-hexylaminocyclohexanoat
(58) 4-Cyanocyclohexyl-4'-pyrrolidinocyclohexanoat
(59) 4-Nitrocyclohexyl-4'-pyrrolidinocyclohexanoat
(60) 4-Bromocyclohexyl-4'-pyrrolidinocyclohexanoat
(61) 4-Propylcyclohexyl-4'-pyrrolidinocyclohexanoat
(62) 4-Pentylcyclohexyl-4'-pyrrolidinocyclohexanoat
(63) 4-Heptylcyclohexyl-4'-pyrrolidinocyclohexanoat
(64) 4-Butylaminocyclohexyl-4'-cyanocyclohexanoat
(65) 4-Butylaminocyclohexyl-4'-nitrocyclohexanoat
(66) 4-Butylaminocyclohexyl-4'-bromocyclohexanoat
(67) 4-Hexylaminocyclohexyl-4'-cyanocyclohexanoat
(68) 4-Hexylaminocyclohexyl-4'-bromocyclohexanoat
(69) 4-Hexylaminocyclohexyl-4'-nitrocyclohexanoat
(70) 4-Pyrrolidinocyclohexyl-4'-cyanocyclohexanoat
(71) 4-Pyrrolidinocyclohexyl-4'-nitrocyclohexanoat
(72) 4-Pyrrolidinocyclohexyl-4'-bromocyclohexanoat
(73) 4-Pyrrolidinocyclohexyl-4'-propylcyclohexanoat
(74) 4-Pyrrolidinocyclohexyl-4'-pentylcyclohexanoat

Alle angeführten Verbindungen liegen in der trans-Konfiguration vor.

## Beispiel 75

Herstellung von 4-Phenylcyclohexyl-4'-n-propylcyclohexanoat (Formel 1 ; X = n—$C_3H_7$, Y = Phenyl)

2,2 g (13 mMol) n-Propylcyclohexancarbonsäure (Formel 2 ; X = n—$C_3H_7$) wurden in 10 ml Thionylchlorid während 2 Std. auf Rückfluss erwärmt, dann eingeengt, zweimal mit je 20 ml Benzol versetzt und wiederum eingeengt, um die letzten Reste Thionylchlorid auszutreiben. 2,2 g (12,5 mMol) 4-Phenylcyclohexanol (Formel 3 ; Y = Phenyl) wurden in 20 ml Pyridin bei Raumtemperatur vorgelegt und tropfenweise unter Kühlung mit dem Säurechlorid in 10 ml Pyridin versetzt. Das Reaktionsgemisch wurde 5 Std. bei Raumtemperatur gerührt, auf Eis/2N HCl gegossen und dreimal mit Aether extrahiert. Die Aetherextrakte wurden einmal mit 2N NaOH/Eis, einmal mit NaCl-Lösung gewaschen, über $Na_2SO_4$ getrocknet und eingeengt. Als Rohprodukt wurden 3,6 g gelbe Kristalle erhalten. Nach dreimaligem Umkristallisieren aus EtOH wurde das Zielprodukt in Form von farblosen Kristallen (GC 99,8 %) erhalten, $T_m$ 84,4 °C ; $T_c$ 69,5 °C.

## Beispiele 76-96

Nach der oben beschriebenen Arbeitsweise wurden in analoger Weise die folgenden Verbindungen der Formel (1) hergestellt, die alle in trans-Konfiguration vorlagen :

(76) 4-Propylcyclohexyl-(4"-propylpiperidino-4')-cyclohexanoat
(77) 4-Pentylcyclohexyl-(4"-propylpiperidino-4')cyclohexanoat
(78) 4-Nonylcyclohexyl-(4"-propylpiperidino-4')-cyclohexanoat
(79) 4-Cyanocyclohexyl-(4"-propylpiperidino-4')-cyclohexanoat
(80) 4-Nitrocyclohexyl-(4"-propylpiperidino-4')-cyclohexanoat
(81) 4-Bromocyclohexyl-(4"-propylpiperidino-4')-cyclohexanoat
(82) 4-Propylcyclohexyl-(4"-propyl-p-phenylen-4')-cyclohexanoat
(83) 4-Propylcyclohexyl-(4"-pentyl-p-phenylen-4')-cyclohexanoat
(84) 4-Propylcyclohexyl-(4"-cyano-p-phenylen-4')-cyclohexanoat
(85) 4-Propylcyclohexyl-(4"-bromo-p-phenylen-4')-cyclohexanoat
(86) 4-Propylcyclohexyl-(4"-nitro-p-phenylen-4')-cyclohexanoat
(87) (4"-Propyl-p-phenylen-4')-cyclohexyl-4-propylcyclohexanoat
(88) (4"-Pentyl-p-phenylen-4')-cyclohexyl-4-propylcyclohexanoat
(89) (4"-Cyano-p-phenylen-4')-cyclohexyl-4-propylcyclohexanoat
(90) (4"-Nitro-p-phenylen-4')-cyclohexyl-4-propylcyclohexanoat
(91) (4"-Bromo-p-phenylen-4')-cyclohexyl-4-propylcyclohexanoat
(92) (4"-Propyl-p-phenylen-4')-cyclohexyl-4-pentylcyclohexanoat
(93) (4"-Pentyl-p-phenylen-4')-cyclohexyl-4-pentylcyclohexanoat
(94) (4"-Cyano-p-phenylen-4')-cyclohexyl-4-pentylcyclohexanoat
(95) (4"-Nitro-p-phenylen-4')-cyclohexyl-4-pentylcyclohexanoat
(96) (4"-Bromo-p-phenylen-4')-cyclohexyl-4-pentylcyclohexanoat

Beispiele 97-105

In der folgenden Tabelle sind weitere Verbindungen der Formel (1) mit der jeweiligen Bedeutung von X und Y und den für anisotrope Verbindungen üblichen Kennwerten angegeben. Die Zahlenwerte in den Spalten zwischen « K » (kristallin), « S » (smektisch), « N » (nematisch) und « I » (isotrop) sind die Temperaturwerte in °C des jeweiligen Ueberganges.

| Beispiel Nr. | X | Y | K | S | N | I |
|---|---|---|---|---|---|---|
| 97 | $C_3H_7$ | $C_3H_7$ | 22.8 | | 36.6 | — |
| 98 | $C_5H_{11}$ | $CH_3$ | 18.3 | | (3.3) | — |
| 99 | $C_5H_{11}$ | $C_3H_7$ | 25.1 | 36.8 | 52.1 | — |
| 100 | $C_5H_{11}$ | CN | 59.8 | | (16*) | — |
| 101 | $C_3H_7$ | 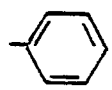 | 84.4 | | (69.5) | — |
| 102 | $C_3H_7$ | 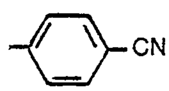 | 103.0 | | 206.1 | — |
| 103 | $C_5H_{11}$ | 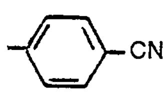 | 94.2 | | 201.3 | — |
| 104 | $C_7H_{15}$ | 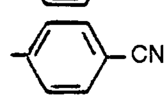 | 104.6 | | 189.2 | — |
| 105 | $C_5H_{11}$ 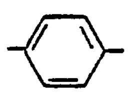 | $C_3H_7$ | 84.2 | 88.6 | 177.3 | — |

* Bestimmung durch Extrapolation.

**Ansprüche**

1. Anisotrope Verbindungen der Formel (1)

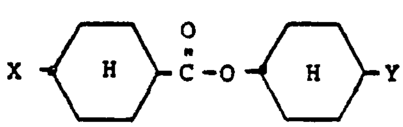 (1)

trans trans

in der X und Y jeweils Alkylgruppen mit 1 bis 12 C-Atomen, Alkoxygruppen mit 1 bis 12 C-Atomen, N-Monoalkylaminogruppen mit 1 bis 12 C-Atomen im Alkylteil, Halogenatome, Nitrilgruppen oder Nitrogruppen oder cyclische Reste der Formeln

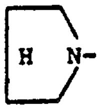 , 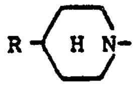 , 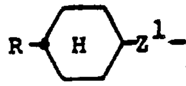 oder 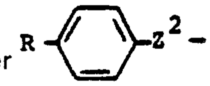

trans

bedeuten, in welcher R das Wasserstoffatom, ein Halogenatom, die Nitril- oder Nitrogruppe oder eine Alkyl- oder Alkoxygruppe mit 1 bis 12 C-Atomen ist, $Z^1$ die Gruppe —COO— oder —OOC— und $Z^2$ gleich $Z^1$ ist oder eine einfache Kovalenzbindung darstellt, mit der Massgabe, dass nur eine der Gruppen X, Y einen cyclischen Rest bedeutet.

2. Flüssigkristallmischung, dadurch gekennzeichnet, dass sie mindestens eine anisotrope Verbindung der Formel (1)

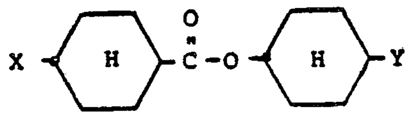

trans trans (1)

enthält, in der X und Y jeweils Alkylgruppen mit 1 bis 12 C-Atomen, Alkoxygruppen mit 1 bis 12 C-Atomen, N-Monoalkylaminogruppen mit 1 bis 12 C-Atomen im Alkylteil, Halogenatome, Nitrilgruppen oder Nitrogruppen oder cyclische Reste der Formeln

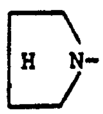 , 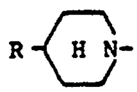 , 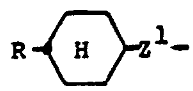 oder 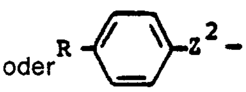

trans

bedeuten, in welchen R das Wasserstoffatom, ein Halogenatom, die Nitril- oder Nitrogruppe oder eine Alkyl- oder Alkoxygruppe mit 1 bis 12 C-Atomen ist, $Z^1$ die Gruppe —COO— oder —OOC— und $Z^2$ gleich $Z^1$ ist oder eine einfache Kovalenzbindung darstellt, mit der Massgabe, dass nur eine der Gruppen X, Y einen cyclischen Rest bedeutet.

3. Verfahren zur Herstellung von anisotropen Verbindungen der Formel (1)

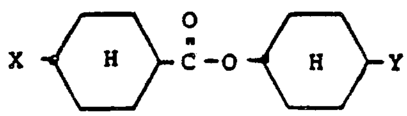

trans trans (1)

in der X und Y jeweils Alkylgruppen mit 1 bis 12 C-Atomen, Alkoxygruppen mit 1 bis 12 C-Atomen, N-Monoalkylaminogruppen mit 1 bis 12 C-Atomen im Alkylteil, Halogenatome, Nitrilgruppen oder Nitrogruppen oder cyclische Reste der Formeln

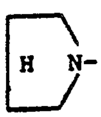 , 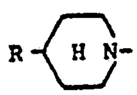 , 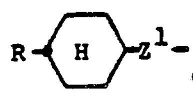 oder 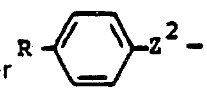

trans

bedeuten, in welchen R das Wasserstoffatom, ein Halogenatom, die Nitril- oder Nitrogruppe oder eine Alkyl- oder Alkoxygruppe mit 1 bis 12 C-Atomen ist, $Z^1$ die Gruppe —COO— oder —OOC— und $Z^2$ gleich $Z^1$ ist oder eine einfache Kovalenzbindung darstellt, mit der Massgabe, dass nur eine der Gruppen X, Y einen cyclischen Rest bedeutet, gekennzeichnet durch die Kondensation einer Cyclohexancarbonsäure der Formel (2)

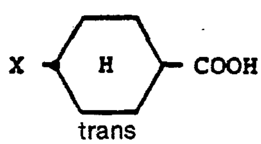

trans (2)

in der X die oben angegebene Bedeutung hat, oder eines funktionellen Derivates dieser Säure, mit einem Cyclohexanol der Formel (3)

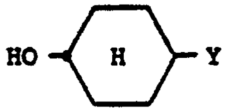

trans

(3)

in der Y die oben angegebene Bedeutung hat.

4. Verfahren nach Anspruch 3, dadurch gekennzeichnet, dass als funktionelles Derivat der Verbindung (2) das entsprechende Säurehalogenid, insbesondere Säurechlorid, verwendet wird.

5. Verfahren nach Anspruch 4, dadurch gekennzeichnet, dass die Kondensation in Gegenwart einer organischen Base, wie Pyridin, durchgeführt wird.

**Claims**

1. Anisotropic compounds of the formula (1)

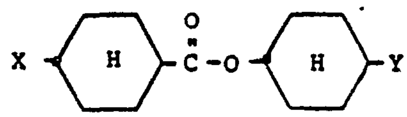

trans trans

(1)

in which X and Y are each alkyl groups having 1 to 12 C atoms, alkoxy groups having 1 to 12 C atoms, N-monoalkylamino groups having 1 to 12 C atoms in the alkyl moiety, halogen atoms, nitrile groups or nitro groups or cyclic radicals of the formulae

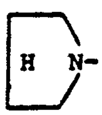 , 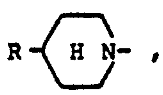 , 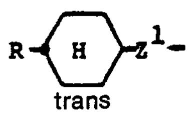 or 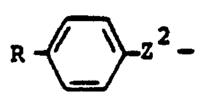

trans

in which R is a hydrogen atom, a halogen atom, a nitrile or nitro group or an alkyl or alkoxy group having 1 to 12 C atoms, $Z^1$ is a group —COO— or —OOC— and $Z^2$ is the same as $Z^1$ or is a single covalent bond, with the proviso that only one of the groups X and Y is a cyclic radical.

2. Liquid crystal mixture, characterised in that it contains at least one anisotropic compound of the formula (1)

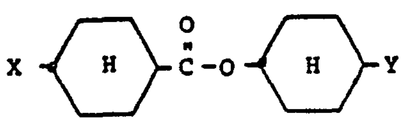

trans trans

(1)

in which X and Y are each alkyl groups having 1 to 12 C atoms, alkoxy groups having 1 to 12 C atoms, N-monoalkylamino groups having 1 to 12 C atoms in the alkyl moiety, halogen atoms, nitrile groups or nitro groups or cyclic radicals of the formulae

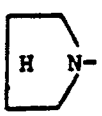 , 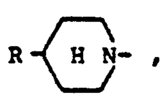 , 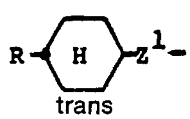 or 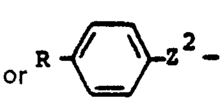

trans

in which R is a hydrogen atom, a halogen atom, a nitrile or nitro group or an alkyl or alkoxy group having 1 to 12 C atoms, $Z^1$ is a group —COO— or —OOC— and $Z^2$ is the same as $Z^1$ or is a single covalent bond, with the proviso that only one of the groups X and Y is a cyclic radical.

3. A process for the preparation of anisotropic compounds of the formula (1)

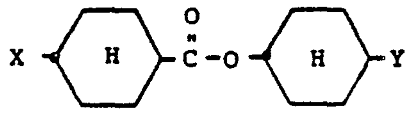
trans trans

(1)

in which X and Y are each alkyl groups having 1 to 12 C atoms, alkoxy groups having 1 to 12 C atoms, N-monoalkylamino groups having 1 to 12 C atoms in the alkyl moiety, halogen atoms, nitrile groups or nitro groups or cyclic radicals of the formulae

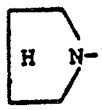 , 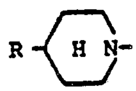 , 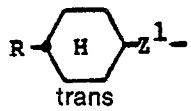 or 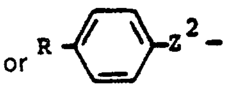

trans

in which R is a hydrogen atom, a halogen atom, a nitrile or nitro group or an alkyl or alkoxy group having 1 to 12 C atoms, $Z^1$ is a group —COO— or —OOC— and $Z^2$ is the same as $Z^1$ or is a single covalent bond, with the proviso that only one of the groups X and Y is a cyclic radical, characterised by condensing a cyclohexanecarboxylic acid of the formula (2)

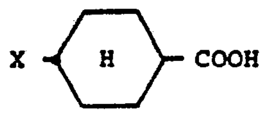
trans

(2)

in which X has the meaning given above, or a functional derivative of this acid, with a cyclohexanol of the formula (3)

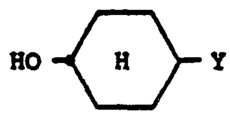
trans

(3)

in which Y has the meaning given above.

4. Process according to Claim 3, characterised in that, as the functional derivative of the compound (2), the corresponding acid halide, in particular the acid chloride, is used.

5. Process according to Claim 4, characterised in that the condensation is carried out in the presence of an organic base, such as pyridine.

**Revendications**

1. Composés anisotropes de formule (1)

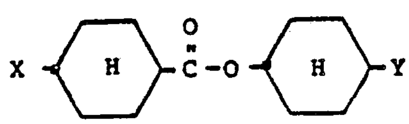
trans trans

(1)

où X et Y représentent chacun des radicaux alcoyle de 1 à 12 atomes de carbone, des radicaux alcoxy de 1 à 12 atomes de carbone, des radicaux monoalcoylamino de 1 à 12 atomes de carbone dans la partie alcoyle, des atomes d'halogène, des radicaux nitrile ou des radicaux nitro ou bien des radicaux cycliques de formule :

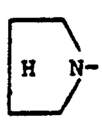 , 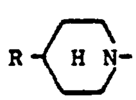 , 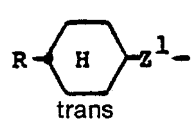 où 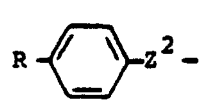

trans

où R représente l'atome d'hydrogène, un atome d'halogène, le radical nitrile ou nitro ou un radical alcoyle ou alcoxy de 1 à 12 atomes de carbone, $Z^1$ représente le radical —COO— ou —OOC— et $Z^2$ est identique à $Z^1$ ou représente une liaison covalente simple, avec la restriction que seul l'un des radicaux X, Y est un radical cyclohexyle.

2. Mélange de cristaux liquides, caractérisé en ce qu'il comprend au moins un composé anisotrope de formule (1)

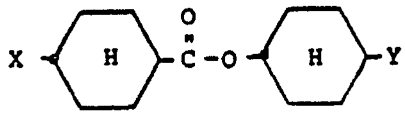

trans trans

(1)

où X et Y représentent chacun des radicaux alcoyle de 1 à 12 atomes de carbone, des radicaux alcoxy de 1 à 12 atomes de carbone, des radicaux N-monoalcoylamino de 1 à 12 atomes de carbone dans la partie alcoyle, des atomes d'halogène, des radicaux nitrile ou des radicaux nitro, ou bien des radicaux cycliques de formule :

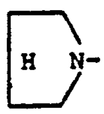 , 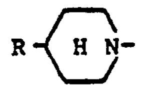 , 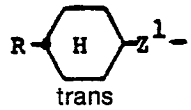 ou 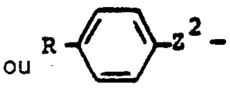

trans

où R représente l'atome d'hydrogène, un atome d'halogène, le radical nitrile ou nitro ou un radical alcoyle ou alcoxy de 1 à 12 atomes de carbone, $Z^1$ représente le radical —COO— ou —OOC— et $Z^2$ est identique à $Z^1$ ou représente une liaison covalente simple, avec la restriction que seul l'un des radicaux X, Y est un radical cyclique.

3. Procédé de préparation de composés anisotropes de formule (1)

(1)

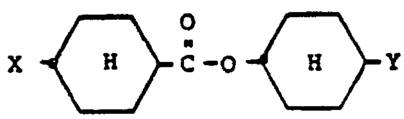

trans trans

où X et Y représentent chacun des radicaux alcoyle de 1 à 12 atomes de carbone, des radicaux alcoxy de 1 à 12 atomes de carbone, des radicaux N-monoalcoylamino de 1 à 12 atomes de carbone dans la partie alcoyle, des atomes d'halogène, des radicaux nitrile ou des radicaux nitro ou bien des radicaux cycliques de formule :

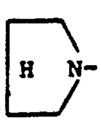 , 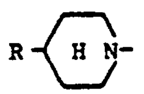 , 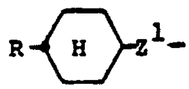 ou 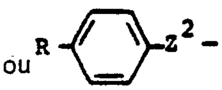

trans

où R représente l'atome d'hydrogène, un atome d'halogène, le radical nitrile ou nitro ou un radical alcoyle ou alcoxy de 1 à 12 atomes de carbone, $Z^1$ représente le radical —COO— ou —OOC— et $Z^2$ est identique à $Z^1$ ou représente une liaison covalente simple, avec la restriction que seul un des radicaux X, Y est un radical cyclique, caractérisé par la condensation d'un acide cyclohexanecarboxylique de formule (2)

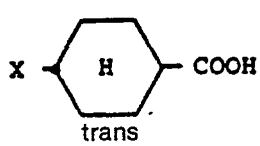

trans

(2)

où X a la signification attribuée ci-dessus, ou d'un dérivé fonctionnel de cet acide, avec un cyclohexanol de formule (3)

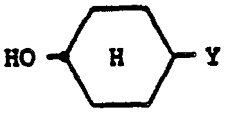

trans

(3)

où Y a la signification attribuée ci-dessus.

4. Procédé suivant la revendication 3, caractérisé en ce qu'on utilise comme dérivé fonctionnel du composé (2) l'halogénure d'acide correspondant, en particulier le chlorure d'acide.

5. Procédé suivant la revendication 4, caractérisé en ce qu'on exécute la condensation en présence d'une base organique comme la pyridine.